# EUROPEAN PATENT SPECIFICATION

(11) **EP 3 074 521 B1**
(45) Date of publication and mention of the grant of the patent: **05.01.2022**
(21) Application number: 14820726.9
(22) Date of filing: 13.11.2014
(51) Int. Cl.: C12P 21/00, C08H 1/06, C08H 1/00, C07K 1/12, A23J 3/32, A23J 1/10, C08L 89/04

(54) **PROCESS FOR THE PREPARATION OF SOLUTIONS OF PROTEIN KERATIN MATERIALS**
VERFAHREN ZUR HERSTELLUNG VON PROTEINKERATINMATERIALLÖSUNGEN
PROCÉDÉ DE PRÉPARATION DE SOLUTIONS DE MATIÈRES PROTÉIQUES KÉRATINIQUES

(30) Priority: 18.11.2013 CZ 20130900
(43) Date of publication of application: 05.10.2016
(73) Proprietor: Tonak A.S., 74101 Nový Jicín (CZ)
(72) Inventor: ANDREE, Dalibor, 74301 Bílovec (CZ); KOLOMAZNÍK, Karel, 76005 Zlín (CZ); PECHA, Jiri, 76005 Zlín (CZ); BARINOVÁ, Michaela, CZ76001 Zlín (CZ); JELÍNEK, Milos, 50008 Hradec Králové (CZ)
(74) Representative: Kreizlova, Dana
(86) International application number: PCT/CZ2014/000134
(87) International publication number: WO 2015/070829

(56) References cited:
- WO-A1-2013/007223
- DE-A1-102011 055 889
- JP-A- H0 251 533
- US-A- 3 904 748
- US-A- 3 970 614
- Pavel Mokrejs ET AL: "Producing Keratin Hydrolysates from Sheep Wool", ORIENTAL JOURNAL OF CHEMISTRY, 5 October 2011 (2011-10-05), pages 1303-1309, XP055056093, Retrieved from the Internet: URL:http://www.orientjchem.org/dnload/Pave l-Mokrejs-Ondrej-Krejci-and-Petr-Svoboda-/ OJCV027I04P1303-1309.pdf [retrieved on 2013-03-12] cited in the application
- Pavel Mokrejs ET AL: "Processing poultry feathers into keratin hydrolysate through alkaline-enzymatic hydrolysis", Waste Management & Research, vol. 29, no. 3, 1 May 2010 (2010-05-01), pages 260-267, XP055515796, DOI: 10.1177/0734242X10370378

## Description

### Technical field

The invention relates to a **method of** preparing solutions of **water soluble** keratin-based protein materials **by hydrolysis of the keratinous protein material with a strong organic base. The method has** various uses in cosmetics, in the production of keratin shampoos and soaps as well as nail varnish removers, packaging for food products, fertilizers, or in the preparation of biodegradable films.

### Background of the invention

Due to its chemical structure containing disulphide bonds, keratin in its natural form (feathers, hair, wool) is insoluble in the majority of common solvents and, unlike collagen, resistant against the action of most proteolytic enzymes [Schrooyen, P. 1999: Feather Keratins : Modification and Film Formation, Ph.D. Thesis, University of Twente, Press: FEBODRUK BV, Enschede, The Nederlands, 1999].

The solubility of keratin materials is determined by cleavage of the disulphide bridges, which is usually made by oxidation or reduction methods or through cleavage by sulphites (sulphitolysis).

The oxidation methods break the disulphide bonds using organic peracids. One of those frequently used is performic acid [Sanger, F. 1949, Biochem. J. 44, p. 126], the reaction of which with keratin produces a series of unstable oxidation intermediates. Cleavage by sulphites converts cystine disulfide bonds into cysteine sulfonate and cysteine [for example: Cole, R. D. 1967, Methods in Enzymology 11, p. 206].

The most widely used methods involve reduction procedures using e.g. thiols or dithiols. The most commonly used thiol used for the disulphide bond cleavage during processing of different types of keratin is 2-mercaptoethanol or dithiothreitol [Schrooyen, P. 1999: Feather keratins: Modification and Film Formation, Ph.D. Thesis, University of Twente, Press: FEBODRUK BV, Enschede, The Nederlands, 1999]; in this case, the efficiency of keratin solution preparation is increased by denaturing additives, e.g. urea [Gregg, K. and Rogers, GE 1986: Composition, Structure and biogenesis. In: Biology of the integument 2 Vertebrates, J. Bereiter-Hahn et al. (eds.), Springer-Verlag, Berlin, p. 666-694].

Alkaline solutions of thioglycolates have been successfully used for the solubilization of keratin material from feathers [e.g. Harrap,B. S. and Woods, E. F. 1964, Biochem. J. 92, 8-18]. In the process of raw hide liming, a mixture of sodium sulfide with calcium hydroxide is used for the removal of hair (dehairing) [Blažej, A. et al. 1984: Technology of hides and pelts, SNTL Prague, 451 pp.]; at higher sulfide concentrations, the said process can be also used for feather solubilization [Schroeder, W. W. et al. 1955, Journal of the American Chemical Society 77, 3901-3908].

A patented method [US Patent No. 3,970,614] describes solubilization of keratin material of various origins (hair, feathers, hooves, etc.) at atmospheric pressure and higher temperatures with N,N-dimethylformamide. However, utilization of this substance is disputable in view of the fact that the International Agency for Research on Cancer (IARC) classifies N,N-dimethylformamide as potential human carcinogen.

Despite the resistance of keratin to the action of most proteolytic enzymes, several methods have been described of microbial and/or enzyme hydrolysis of keratin materials of various origins [e.g. US Patent No. 5,395,613, or US patent applications No. 2006134092 and 2010/0196302]. The advantage of these methods resides in milder reaction conditions; nevertheless, biotechnological treatment of keratin also entails risks. One of the disadvantages of microbial degradation is frequent utilization of pathogenic or dermatolytic strains of bacteria (*Bacillus* sp., etc.) or molds (*Aspergillus* sp.) and the consequent need for seeking non-pathogenic organisms producing keratinases of comparable efficiency. Refining of the obtained crude products is therefore challenging and the hydrolytic procedures are often unsatisfactory in terms of economic viability as well as environmental protection. Besides the above stated biotechnological processes, several methods of keratin material pretreatment have been patented [see the US Patents No. 5,262,307 and 4,390,525 or Canadian Patent No. 1108542], which precede the actual processing of keratin materials by some of the methods discussed above.

Patent documents and other literary sources also involve methods of the processing of keratin materials of various origins by a combination of alkaline hydrolysis using a strong inorganic base (KOH, NAOH, or in combination with Sr(OH)2) and subsequent hydrolysis with a proteolytic enzyme/enzymes [Mokrejš et al., 2010, Waste Management & Research 29(3), 260-267; Mokrejš et al. 2011, Oriental Journal of Chemistry 27(4), 1303-1309; US Patent No. 5262307 ]. From the most recent resources, Gupta et al. [Gupta et al. 2012, J. Chem. Chem. Eng 6, 732-737] describe a process of a chemical extraction of keratin from poultry feathers with potassium cyanide, thioglycolic acid and sodium sulfide.

Main disadvantages of the above mentioned methods of hydrolysis or solubilization of keratin materials are low yield of hydrolyzed keratin protein, relatively expensive chemicals involved in the processes, and most importantly, high ash content resulting in low quality of the products, especially when using reduction processes. For the specific purposes of soluble keratin applications, challenging separation and refining of the final soluble keratin product must be carried out.

### Summary of the invention

The above mentioned drawbacks of the existing procedures are eliminated by the newly developed method of preparing solutions of keratin-based protein materials according to the invention. The invention **is set out in the appended set of claims.**

### Description of the invention

The invention resides in the fact that the keratinous protein material is hydrolyzed using a strong organic base, or a mixture of organic bases or their solutions provided that the resulting product obtained by said alkaline conditions is water soluble.

At least one amine the boiling point of which at normal pressure is below 200 °C can be used with advantage as a strong organic base, primarily at least one amine from the group comprising cyclohexylamine, isopropylamine, diisopropylamine, butylamine and diethylamine.

It is advantageous from the technological point of view to apply said strong organic bases in the form of solutions in suitable solvents, especially in water, while the minimal pH value of the aqueous solution equals 11.

The process of dissolving the keratin material runs in either one or two steps. In the first step, which is carried out at a temperature at 100 °C (dependent on the choice of the organic base or a mixture of organic bases), or at the boiling point of the reaction mixture, substantial destruction of the keratin protein occurs at high pH value (pH 11-12); after termination of this step, considerable part of the used organic base is distilled off, resulting in the reduction of the pH value to 8-9. A keratin hydrolyzate solution is obtained as well as solid, unreacted part. The yield of said procedure, according to any of the claims 1-4, is high, namely around 70 %. The obtained keratin hydrolyzate is of high quality, with low ash content. Part of the used organic base is present in the hydrolyzate in the form of salts with free carboxyl groups of the hydrolyzate.

After the first phase it is preferable to further process the solid, unreacted part by the following procedure. The solid part is subjected to enzymatic hydrolysis catalyzed by a proteolytic enzyme dosed with advantage in the amount of 0.2-5 % (related to the original weight of the input keratin material) provided that the reaction temperature is selected according to the type of enzyme. After termination of the enzymatic hydrolysis, the residue of the organic base or a mixture thereof is distilled off. The said hybrid two-step procedure according to any of claims 5 - 7 makes it possible to achieve more than 95% yield of the soluble keratin hydrolyzate. It is also possible to process in a similar way the entire reaction mixture from the first step.

In practical application of the above-described method it is advantageous that the organic base, or a mixture of organic bases, is to considerable extent replaced with a condensate containing a recycled organic base or a mixture of organic bases, which was formed by regeneration of the organic base or a mixture of organic bases previously used in the process.

The main advantage of the use of said organic base is a high yield of soluble keratin material, high purity of the product, which is given by very low ash content, and the economy of the invented technology, because more than 80 % of volatile organic bases can be recovered by distillation, thereby substantially reducing the operating costs.

### Example embodiments of the invention

### Example 1

15 g of rabbit hair containing 86 % of dry matter, 13.5 % of nitrogen and 3.5 % of ash (related to the dry matter content) was mixed with 36 g (50 ml) of pure isopropylamine and 50 ml of water; the mixture was heated for 2 hours under a reflux condenser at the boiling point temperature (70 °C). After the elapse of said time, 50 ml were distilled off the reaction mixture and the distillation residue was filtered. The filtrate was dried at room temperature yielding 9.7 g of non-enzymatic keratin hydrolyzate.

### Example 2

Proceeding as in Example 1, except that an 80% mixture of n-butylamine and isopropylamine was used instead of pure isopropylamine. The reaction mixture was again kept at its boiling point. After distilling off 50 ml from the reaction mixture, the reaction mixture was filtered, the filtrate was dried freely and the yield was 10.1 g of non-enzymatic hydrolyzate containing (as in the previous case) used alkyl amines bound to free carboxylic groups.

### Example 3

15 g of waste sheep wool together with 50.3 g of cyclohexylamine and 125 ml of water was heated at the reaction mixture boiling point (99 °C) for 4 hours. After that, 120 ml of cyclohexylamine aqueous solution was distilled off. The distillate contained 35 % of pure cyclohexylamine. The distillation residue was filtered and both the filtrate and the filter cake were dried. The procedure yielded 11.1 g of non-enzymatic dry water-soluble keratin product and 5.5 g of dry filter cake.

### Example 4

60 g of waste rabbit hair produced during the manufacture of hats and containing 88 % of dry matter, 14 % of nitrogen and 3 % of ash (related to the dry matter content) was placed in a stirred reactor together with 500 ml of water and 200 g of cyclohexylamine. The reaction mixture was kept under continuous stirring at the boiling point temperature (99 °C) for 4 hours. The subsequent distillation gave 600 ml of distillate containing 29 % of cyclohexylamine (i.e. 174 g thereof).

After the distillation residue has been cooled, 120 ml of water and 0.8 g of proteolytic enzyme (Alcalase DX-L, manufactured by Novo Nordisk, Denmark) were added and the reaction mixture was maintained under continuous stirring at a temperature of 70 °C for 2 hours. After termination of the enzymatic hydrolysis, the reaction mixture was filtered; the filtrate was concentrated in a vacuum evaporator and dried at 105 °C. The procedure gave 61 g of dry keratin hydrolyzate and 4.3 g of dry filter cake with an ash content of 3.7 %.

60 g of keratin raw material represent about 51 g of keratin protein; the yield was 61 g of pure keratin hydrolyzate, i.e. 10 g of cyclohexylamine is bound to carboxylic groups of the hydrolyzate and 16 g of cyklohehylamine represent a loss by evaporation (8 %), when performing the above-described procedure.

### Example 5

300 g of rabbit hair containing 88 % of dry matter, 14 % of nitrogen and 3 % of ash (related to the dry matter content) was mixed with 1 kg of cyclohexylamine and 5.52 kg of demineralized water. The mixture was heated at the boiling point (97 °C) for 6 hours. Immediately afterwards the excess cyclohexylamine was distilled off, yielding 1.93 kg of distillate with a 38.4% content of cyclohexylamine. The said distillate was then used as the regenerated organic base for the following Example 6.

The distillation residue (1.85 kg) was filtered, giving 1.56 kg of filtrate with dry matter content of 19.3 %, which was subsequently concentrated by evaporation at 105 °C to dry matter content of 95 %. The filter cake, the weight of which was 0.29 kg, was placed in a hydrogenation reactor and 0.2 kg of demineralized water together with 25 g of proteolytic enzyme (Alcalase DX-L) was added. The enzymatic hydrolysis was carried out at a temperature of 70 °C and pH of 9.5 for 3.5 hours. After that the reaction mixture was filtered, yielding 0.176 kg of filter cake with moisture content of 36 % and 0.16 kg of filtrate with dry matter content of 12.5 %, which was subsequently concentrated by evaporation of water to a dry matter content of 95 %.

### Example 6

300 g of waste rabbit hair containing 88 % of dry matter, 14 % of nitrogen and 3 % of ash (related to the dry matter content) was mixed with 1.9 kg of distillate containing 38.4 % of cyclohexylamine (regenerated organic base from Example 5) and 258 g of pure cyclohexylamine together with 0.7 kg of demineralized water was added to said mixture. The subsequent procedure was identical to that described in Example 5, yielding a total of 0.25 kg of non-enzymatic hydrolyzate and 26 g of enzymatic hydrolyzate with dry matter content of 95 % and 63 g of non-degraded residue containing soluble enzymatic hydrolyzate, which can be washed out.

## Claims

1. A method of preparing solutions of water soluble keratin-based protein materials by hydrolysis of the keratinous protein material with a strong organic base, **character ised in that** the hydrolysis of keratin-based protein materials is carried out at 100°C or at the boiling point of the reaction mixture at a pH of 11 - 12.

2. The method of claim 1, **wherein** the strong organic base is at least one amine with a boiling point of below 200°C.

3. The method of claim 2, **wherein** the strong organic base is at least one amine selected from the group comprising cyclohexylamine, isopropylamine, diisopropylamine, butylamine and diethylamine.

4. The method of claim 1, further comprising distilling off the organic base(s) until the pH is reduced to 8 - 9, followed by regenerating and recycling at least part of the organic base(s) back to the hydrolysis step.

5. The method of claims 1 - 3, further comprising enzymatic hydrolysis of the entire reaction mixture.

6. The method of claim 4, further comprising enzymatic hydrolysis of the distillation residue, or filtration and enzymatic hydrolysis of the distillation residue.

7. The method of claims 5 and 6, **wherein** the enzymatic hydrolysis is conducted using a proteolytic enzyme dosed in the amount of 0.2 - 5 % related to the original weight of the keratinous material.

## Patentansprüche

1. Verfahren zur Herstellung von Proteinkeratinmateriallösungen durch Hydrolyse des Proteinkeratinmaterials mit einer starken organischen Base, **dadurch gekennzeichnet, dass** die Hydrolyse von Proteinmaterialien auf Keratinbasis bei 100°C oder am Siedepunkt des Reaktionsgemisches bei einem pH-Wert von 11 - 12 durchgeführt wird.

2. Verfahren nach Anspruch 1, **wobei** es sich bei der starken organischen Base um mindestens ein Amin mit einem Siedepunkt von unter 200°C handelt.

3. Verfahren nach Anspruch 2, **wobei** die starke organische Base mindestens ein Amin ist, ausgewählt aus der Gruppe, die Cyclohexylamin, Isopropylamin, Diisopropylamin, Butylamin und Diethylamin umfasst.

4. Verfahren nach Anspruch 1, **bei dem** man die organische(n) Base(n) abdestilliert, bis der pH-Wert auf 8 - 9 gesenkt ist, und anschließend mindestens einen Teil der organischen Base(n) regeneriert und in den Hydrolyseschritt zurückführt.

5. Verfahren nach Anspruch 1 bis 3, **das** die enzymatische Hydrolyse des gesamten Reaktionsgemisches ferner umfasst.

6. Verfahren nach Anspruch 4, **ferner umfassend** die enzymatische Hydrolyse des Destillationsrückstandes oder die Filtration und enzymatische Hydrolyse des Destillationsrückstandes.

7. Verfahren nach Anspruch 5 und 6, **wobei** die enzymatische Hydrolyse unter Verwendung eines proteolytischen Enzyms durchgeführt wird, das in einer Menge von 0,2 bis 5 % dosiert wird, bezogen auf das ursprüngliche Gewicht des keratinischen Materials.

## Revendications

1. Procédé de préparation de solutions de matiéres protéiques kératiniques soluble dans l'eau par hydrolyse du matiéres protéique kératiniques avec une forte base organique, **caractérisée par le fait que** l'hydrolysise des matériaux protéiques basés sur de la kératine est portée à 100°C (212 °F) ou au point d'ébullition du mélange en réaction à un pH de 11 - 12.

2. Procédé selon la revendication 1, **dans lequel** le fait que au moins un amine dans la forte base organique avec un point d'ébullition au-dessous de 200°C (392 °F).

3. Procédé selon la revendication 2, dans **lequel** le fait que au moins un amine dans la forte base organique comprenant de la cyclohexylamine, de l'isopropylamine, de la diisopropylamine, de la butylamine et de la diéthylamine.

4. Procédé selon la revendication 1, **dans lequel** le fait que comprenant de plus la distillation de la base organique ou des bases organiques jusqu'à ce que le pH soit réduit à 8 - 9, suivi par la régénération et le recyclage d'au moins une partie de la base organique ou des bases organiques pour revenir à l'étape de l'hydrolyse.

5. Procédé selon la revendications 1-3, **dans lequel** le fait que comprenant de plus l'hydrolyse enzymatique de tout le mélange en réaction.

6. Procédé selon la revendication 4, **dans lequel** le fait que comprenant de plus l'hydrolyse enzymatique du résidu de distillation ou le filtrage et l'hydrolyse enzymatique du résidu de distillation.

7. Procédé selon la revendications 5 et 6, **dans lequel** le fait que l'hydrolyse enzymatique est menée à bien en utilisant une enzyme protéolytique en quantité de 0.2 - 5 % par rapport au poids original du matériau kératineux.
